# EUROPÄISCHE PATENTSCHRIFT

(11) **EP 3 451 944 B1**
(45) Veröffentlichungstag und Bekanntmachung des Hinweises auf die Patenterteilung: **15.09.2021**
(21) Anmeldenummer: 17721623.1
(22) Anmeldetag: 02.05.2017
(51) Int. Cl.: A61B 17/15

(54) **FIBULA-KNOCHENMATERIALENTNAHME- UND -VERBRINGSCHABLONE**
FIBULA BONE MATERIAL REMOVAL AND TRANSFER TEMPLATE
GABARIT DE PRÉLÈVEMENT ET DE TRANSFERT DE SUBSTANCE OSSEUSE PÉRONÉENNE

(30) Priorität: 06.05.2016 DE 102016108426
(43) Veröffentlichungstag der Anmeldung: 13.03.2019
(73) Patentinhaber: Karl Leibinger Medizintechnik GmbH & Co. KG, 78570 Mühlheim (DE)
(72) Erfinder: HERZOG, Rebecca, 78570 Mühlheim (DE); GABELE, Lorenz, 78570 Mühlheim (DE)
(74) Vertreter: Winter, Brandl - Partnerschaft mbB
(86) Internationale Anmeldenummer: PCT/EP2017/060432
(87) Internationale Veröffentlichungsnummer: WO 2017/191139

(56) Entgegenhaltungen:
- WO-A1-2004/039266
- US-A1- 2013 296 872

## Beschreibung

Die Erfindung betrifft eine Fibula-Knochenmaterialentnahme- und - verbringschablone nach dem Oberbegriff des Anspruchs 1.

Aus dem Stand der Technik sind bereits Sägeschablonen, wie Unterkieferresektionsschablonen, bekannt. So offenbart bspw. die WO 2004/039266 A1 eine Sägeschablone, die an einer Fibula oder einer Mandibula einsetzbar ist. Ähnliche Vorrichtungen sind auch aus der US 2013/296872 A1, der US 2012/0029646 A1, der US 2013/0338779 A1 und der US 2013/0304075 A1 bekannt.

Es kommt vor, dass bspw. menschliche Unterkieferknochen, wie eine Mandibula, durch einen Unfall oder karzinogene Veränderungen derart beschädigt sind, dass Teile dieser Knochen entnommen werden müssen. Der Knochen ist danach wieder zu vervollständigen. In jüngster Zeit wurde dazu übergegangen, Knochenabschnitte, die bspw. einer Fibula, also einem Wadenbein, entnommen wurden, wieder an die defektbefreite Stelle / Leerstelle des Unterkiefers einzusetzen. Dabei besteht Bedarf, präzise Schnitte in vorbestimmter Weise sowohl am Unterkieferknochen, als auch dann, dazu korrespondierend, am Wadenbein durchzuführen.

Hierfür werden üblicherweise Unterkieferresektionsschablonen eingesetzt, die für eine präzise Schnittführung am Unterkiefer zuständig sind, genauso wie Fibula-Knochenmaterialentnahme- und -verbringschablonen, die für präzise Schnitte am Wadenbein zuständig sind und gleichzeitig für ein präzises Verbringen der ausgeschnittenen Knochen zum Implantieren im Kieferbereich eingesetzt sind.

Die bisher bekannten Lösungen sind nicht ausreichend präzise, obwohl sie schon sehr umständlich zu handhaben sind. Auch sind sie relativ kostenintensiv. Hier gilt es eine Verbesserung zu erreichen.

Es ist also die Aufgabe der vorliegenden Erfindung, die Nachteile aus dem Stand der Technik abzustellen oder zumindest zu mildern und eine Fibula-Knochenmaterialentnahme- und -verbringschablone zur Verfügung zu stellen, die so eingesetzt werden kann, dass ein ästhetisch und mechanisch ansprechendes Ergebnis im Unterkieferbereich erzielt werden kann, insbesondere unter Nutzung einer verbesserten Unterkieferresektionsschablone.

Vereinzelt wird für Unterkiefer eine spezielle patientenspezifische Schablone angefertigt. Dies ist aber sehr kostenintensiv und zeitaufwändig, weswegen es der Ansatz der vorliegenden Erfindung ist, eine universelle Unterkieferresektionsschablone zur Verfügung zu stellen, die letztlich angepasst ist auf einen repräsentativen Patienten aus der Masse aller potenziellen Patienten.

Es sollen ergo in einem Nebenaspekt insbesondere universelle Schablonen zur Resektion des Unterkiefers aber in einem Hauptaspekt der Fibula - bei anstehender Unterkieferrekonstruktion des mikrovaskulären Fibula-Transplantats sowie ein auf diese Technik abgestimmtes System an Miniplatten zur Transplantatfixierung - zur Verfügung gestellt werden.

Diese Aufgabe wird bei einer gattungsgemäßen Fibula-Knochenmaterialentnahmeund -verbringschablone durch den Gegenstand des Anspruchs 1 gelöst. Damit ist eine Fibula-Knochenmaterialentnahme- und -verbringschablone mit einem Mittelteil, das einen Zentralkörper besitzt, an dessen Enden je ein Knochentrennwerkzeugführabschnitt vorhanden ist, beansprucht, wobei zumindest einer der Knochentrennwerkzeugführabschnitte, vorzugsweise beide Knochentrennwerkzeugführabschnitte, vom Mittelteil wegbewegbar, insbesondere entfernbar oder heranschiebbar gelagert ist/sind. Insbesondere Dreh- und Schwenklagerungen könnten sich hier anbieten. Ziel ist es eine solche Lagerung zu erhalten, dass die Gehrungsflächen der Knochenabschnitte zum Einbau im bspw. Unterkieferbereich aufeinander in Stoß gebracht werden. Ferner ist, etwa in der Mitte des Mittelteils, eine Bügelaufnahmevorrichtung, wie eine Klammer, vorhanden, in die ein Entnahmehilfsbügel eingesteckt ist. Die einzelnen Bestandteile der Fibula-Knochenmaterialentnahme- und -verbringschablone können dann zueinander im Raum unveränderbar festgelegt werden.

Eine solche schon wesentlich verbesserte Fibula-Knochenmaterialentnahme- und - verbringschablone kann auch noch weiter verbessert werden. Diese weiterführenden Verbesserungen werden in den Unteransprüchen beansprucht und nachfolgend näher erläutert.

So ist es von Vorteil, wenn der Knochentrennwerkzeugführabschnitt einen zwischen zwei Vertikalflächen ausgebildeten Führungsschlitz besitzt, der auf dessen Vorderund Rückseite offen ist. Auf diese Weise wird bei der Entnahme von Knochen aus dem Wadenbein ein Verlaufen des Knochentrennwerkzeuges verhindert. Eine präzise Entnahme wird dadurch sichergestellt.

Es ist für das Handling zuträglich, wenn der Führungsschlitz auf dessen Unterseite und/oder Oberseite offen ausgestaltet ist.

Die Einstellbarkeit und Flexibilität im Einsatz / während der Operation wird verbessert, wenn von dem zum Mittelteil separaten Knochentrennwerkzeugführabschnitt ein Balken absteht, der in einer ihn umfassenden Führungsbahn entlang ihrer Längsrichtung verschiebbar gelagert ist.

Zweckmäßig ist es auch, wenn in die Führungsbahn eine Fixierschraube ragt, die zum Befestigen des Balkens ausgelegt ist. Intraoperativ kann dann eine einfache Veränderung durchgeführt werden oder zumindest präoperativ vorbereitet sein.

Es ist von Vorteil, wenn in der Bügelaufnahmevorrichtung ein Loch, wie ein Sackloch oder ein Durchgangsloch, vorhanden ist, das zum arretierenden Aufnehmen eines entnahmehilfsbügelfesten Federabschnittes vorbereitet ist. Ein schnelles Einsetzen und Entfernen des Entnahmehilfsbügels wird dann möglich.

Es hat sich auch bewährt, wenn beiderseits der Klammer ein Aufnahmeloch für eine Knochenschraube vorhanden ist.

Es ist für eine gute Anbringbarkeit zuträglich, wenn das Aufnahmeloch eine Symmetrieachse besitzt, die quer, vorzugsweise senkrecht, zu einer den Zentralkörper aufnehmenden Zentralebene ausgerichtet ist.

Es ist auch für einen flexiblen operativen Einsatz zuträglich, wenn auf einer oder jeder der beiden Knochentrennwerkzeugführabschnitte ein Ergänzungsbauteil auf der zentralkörperabgewandten Seite vorhanden ist. Ferner ist es von Vorteil, wenn an dem distalen Ende des Ergänzungsbauteils jeweils ein weiterer Knochentrennwerkzeugführabschnitt verschiebbar und vom Ergänzungsbauteil entfernbar, angeordnet ist.

Ein vorteilhaftes Ausführungsbeispiel ist auch dadurch gekennzeichnet, dass die Knochentrennwerkzeugführabschnitte des Ergänzungsbauteils den Knochentrennwerkzeugführabschnitten des Zentralkörpers identisch oder zumindest ähnlich sind.

Ein vorteilhaftes Ausführungsbeispiel kann auch so ausgestaltet sein, dass an der am distalen freien Ende des Ergänzungsbauteils vorhandenen Knochentrennwerkzeugführabschnittsausgestaltung zwei Führungsstege vorhanden sind, die in einem gemeinsamen Führungsblock in eigenen Öffnungen geführt sind.

Wenn in dem Führungsblock eine, zwei oder mehr nur den oberen Führungssteg berührende Feststellschraube(n) eingesetzt ist/sind, so kann ein gut zugänglicher Bereich für das Arretieren und Lösen der Arretierung gewählt / genutzt werden. Die Feststellschraube ist vorzugsweise nicht abnehmbar.

Es hat sich auch bewährt, wenn an einem oder beiden Führungsstegen eine Rasterung oder Kerbung vorhanden ist, insbesondere auf der Frontseite mit Rippen, die quer, vorzugsweise orthogonal, zur Längsrichtung eines Führungssteges oder beider Führungsstege verlaufen. Eine haptische Rückkoppelung an den Operateur ist dann relativ einfach realisierbar.

Es ist von Vorteil, wenn an dem Ergänzungsbauteil eine Bügelaufnahmevorrichtung zum arretierenden Aufnehmen des Entnahmehilfsbügels vorhanden ist. Um eine bessere Übersicht für die Segmentaufnahme auf die Bügel zu haben, sind die Segmente mit "R" für Rechts und "L" für Links beschriftet.

Ein vorteilhaftes Ausführungsbeispiel ist auch dadurch gekennzeichnet, dass die Bügelaufnahmevorrichtung des Ergänzungsbauteils identisch oder zumindest ähnlich zur Bügelaufnahmevorrichtung des Zentralkörpers ausgebildet ist.

Es hat sich auch bewährt, wenn die beiden Ergänzungsbauteile zu einer mittig durch den Zentralkörper und von dieser senkrecht durchdrungenen Symmetrieebene spiegelsymmetrisch ausgebildet sind.

Es ist von Vorteil, wenn der Entnahmehilfsbügel zumindest zwei oder vier (90°-) Stufen besitzt. Eine Deformation von Weichgewebe wird dadurch vermieden.

Es ist für die Erfindung zuträglich, wenn die Rückseite der Schablone zum Kontaktieren eines (menschlichen) Wadenbeins vorbereitet ist.

Es ist wünschenswert, wenn die beiden Führungsschlitze am Zentralkörper einen 60°-Winkel +/- 5°.und die beiden Führungsschlitze des Ergänzungsbauteils in Richtung der Rückseite einen spitzen Winkel einschließen, etwa einen ca. 72°-Winkel +/- 5°.

Von Vorteil ist es dabei, wenn die Bügelaufnahmevorrichtungen zum Aufnehmen eines starren /steifen / un-elastischen / formstabil (ähnlich einem Stahlbauteil) Implantierhilfsbügels vorbereitet sind. Dadurch können die einzelnen Knochenstücke positionstreu verlagert werden.

Wenn der Implantierhilfsbügel dem Entnahmehilfsbügel identisch oder ähnlich ist, sich aber in der dem Ergänzungsbauteil und dem Zentralkörper relativ zueinander aufgezwungenen Lage unterscheidet, ergo eine geometrisch an den Anbindungsstellen andere Ausgestaltung hat, so lässt sich ein Überführen der Knochen, die dem Wadenbein entnommen wurden, effizient zum Unterkiefer mit seinen dortigen Lücken realisieren.

Ferner ist es von Vorteil, wenn der Entnahmehilfsbügel das Ergänzungsbauteil und den Zentralkörper in eine gemeinsame Ebene zwingt, jedoch der Implantierhilfsbügel das Ergänzungsbauteil und den Zentralkörper in eine U-Form und/oder eine unterkieferkonturkonforme Ausrichtung zwingt.

Die Fibula-Knochenmaterialentnahme- und -verbringschablone kann auch als Fibula-Resektionsschablone bezeichnet werden und ist auf die durchschnittliche Form der Fibula angepasst. Sie ist also nicht patientenspezifisch, sondern an den durchschnittlichen Patienten angepasst.

Die einzelnen Segmente und Längen sind auf die Unterkieferresektionsschablone abgestimmt. Die Skalen der beiden Schablonen sollten einheitlich gestaltet und aufeinander abgestimmt sein. Eine Berücksichtigung der präoperativen Planung hat jedoch nicht zwingend zu erfolgen.

Mit einem abnehmbaren Bügel werden die einzelnen Segmente miteinander verbunden. Der Bügel soll sowohl von oben als auch von unten anbringbar sein, um die Schablone für die rechte und linke Fibula gleichermaßen zu verwenden. Darüber hinaus soll der Bügel mit einer kleinen Stufe versehen werden, um weiter nach vorne abzustehen, da dieser Bereich häufig durch Weichgewebe behindert wird. Des Weiteren ist der abnehmbare Bügel so gestaltet, dass er elastisch ist. D.h. die einzelnen Segmente schmiegen sich der Fibula an, so dass die Trennschnitte / Sägeschnitte exakt im Winkel ausgeführt werden können.

Die Stellschrauben zur Arretierung der flexiblen Wegeschlitze sollen vorzugsweise orthogonal zur Schablone angebracht werden.

Zur Durchführung der Resektion soll eine oszillierende Säge verwendet werden. Es ist angedacht, den Führungsschlitz / Sägeschlitz ungefähr 1,0 mm breit nach unten oder oben oder beidseitig offen oder geschlossen zu gestalten. Eine seitliche Führung des Sägeblattes sollte jedoch in allen Fällen stattfinden.

Die Fixierung der Schablone an der Fibula erfolgt mit Standardschrauben, die einen Außendurchmesser von ca. 2,0 mm besitzen. In jedem Segment befinden sich zwei Bohrungen für Fixierungen.

Die Schablone sollte bestmöglich eine Fixierung der resezierten Fibula-Segmente mittels Implantate von vorne ermöglichen. Daher ist eine Aussparung an jedem Segment vorgesehen. Gegenwärtig wird das rechte und linke Segment jeweils durch eine im Winkel von ca. 120° angebogene Platte mit dem vorderen Segment verbunden. Die Platte wird von oben angebracht.

Miniplatten mit einem Profil von 1,0 mm, die eine einfache Transplantatfixierung ermöglichen, und anhand der durchschnittlichen Form des Unterkiefers bzw. Fibula-Transplantat bis zu dreidimensional vorgeformt sind, haben sich bewährt. Die Kontur des Durchschnittsunterkiefers sowie der Fibula werden auf Basis repräsentativer Datensätze generiert.

Die Platten müssen in unterschiedlichen Formen und Ausführungen zur Verfügung gestellt werden. Ziel ist es, mit so wenig wie möglich Platten auszukommen. Die exakten Varianten müssen zusammen dann noch ermittelt werden. Denkbar sind jedoch Vier-Lochplatten mit Steg und/oder Sechs-Lochplatten mit Steg. Die Platten sollten über multidirektionale winkelstabile Plattenlöcher verfügen, um sowohl verblockend versorgen zu können, als auch mit Standardschrauben zusammen zu wirken. Je nach Notwendigkeit können spezielle Instrumente zur Fixierung der Platte, bspw. Schraubendreher eingesetzt werden.

Die erfindungsgemäße Fibula-Knochenmaterialentnahme- und - verbringschablone wird verwendet im Zusammenhang mit einer Unterkieferresektionsschablone, mit einem Zentralbauteil, das zum Anbringen an einem Segment, etwa einem Symphysesegment eines Kieferknochens vorbereitet ist, wobei an dem Zentralbauteil mindestens zwei Trennwerkzeugführabschnitte / Sägeblattführabschnitte vorhanden sind, wobei die Verbesserung unter anderem darin gesehen wird, dass zwischen den beiden Trennwerkzeugführabschnitten / Sägeblattführabschnitten eine Positionierhilfe vorhanden ist, um eine raumkorrekte Ausrichtung der Unterkieferresektionsschablone zum Kieferknochen zu bewerkstelligen. Auf diese Weise kann nämlich die Unterkieferresektionsschablone exakter und problemloser als bisher am Schädelknochen, insbesondere einem Kieferknochen, bevorzugt dem Unterkieferknochen des spezifischen Patienten, befestigt werden, sodass eine hochpräzise Entnahme der zu entfernenden Knochenabschnitte durchgeführt werden kann.

Im Rahmen des erfindungsgemäßen Projektes wurden ergo auch universelle Schablonen zur Resektion des Unterkiefers und der Fibula entwickelt. Die Intention war nicht nur, eine deutliche Vereinfachung und Standardisierung des klinischen Eingriffs der Unterkieferrekonstruktion mittels mikrovaskulärem Fibula-Transplantats zu erreichen, sondern auch eine Kosten- und Zeitersparnis gegenüber patientenspezifischen Resektionsschablonen zu erzwingen. Dies wurde durch die erfindungsgemäße Ausgestaltung erreicht.

Zusätzlich wurde zu den Resektionsschablonen auch eine speziell auf diese Technik abgestimmte Plattensystemausgestaltung zur Transplantatfixierung erarbeitet. Entgegen dem aktuellen Stand der Wissenschaft, nachdem zur Überbrückung eines Defekts oder zur Fixierung des Transplantats Rekonstruktionsplatten eingesetzt werden, werden nun wesentlich dünnere Miniplatten mit einer Profilstärke von 1 mm (1,0 mm) eingesetzt, da diese entsprechend den gewonnenen Erfahrungen einen deutlichen Vorteil bei der Materialentfernung bringen.

Platten, wie etwa Rekonstruktionsplatten (größer 1mm dick, bis ca. 3,0 mm) oder Miniplatten (kleiner gleich 1mm dick), die fachwerkartig ausgebildet sein können, werden auf die durchschnittliche Form des Unterkiefers angepasst bzw. auf das nach dem Trennschnitt / Sägeschnitt der Resektionsschablone entstehende Fibula-Transplantat vorgeformt. Dadurch wird eine hohe Passgenauigkeit erreicht und die Transplantatfixierung deutlich erleichtert.

Durchaus bedeutsam ist, dass die Unterkieferresektionsschablone auf die durchschnittliche Form des Unterkiefers angepasst ist. Die Resektionsschablone ist dabei so flexibel wie möglich in ihrer Einstellung, um eine Vielzahl an unterschiedlichen Resektionsmustern, in jedem Fall aber die folgenden Fälle, abzudecken: Drei-Segmentresektion (rechte Unterkieferkörper-Vordersegment-linker Unterkieferkörper), Zwei-Segmentresektion (rechter Unterkieferkörper inkl. vorderem Segment), Zwei-Segmentresektion (linker Unterkieferkörper inkl. vorderem Segment) und Ein-Segmentresektion. Auf diese Weise sind ca. 80 % aller theoretisch möglichen Resektionen abgedeckt.

Im hinteren Bereich der Resektionsschablone wird eine Längenanpassung möglich gemacht, die es erlaubt, den Führungsschlitz / Sägeschlitz je nach Ausmaß des Defekts zu verschieben. Der flexible Führungsschlitz / Sägeschlitz wird mit einer basal angebrachten Feststellschraube arretiert. Die Feststellschraube ist vorzugsweise nicht abnehmbar, um das Handling zu erleichtern und die Unfall-/ Verlustgefahr zu verringern. Allerdings ist es in puncto Aufbereitung der Resektionsschablone vorteilhaft, wenn die Einzelteile leicht demontierbar sind und/oder entstehende Spalte so groß sind, das bei einer Desinfektion genutzte Medium einfach und wirkungsvoll reinigend wirken kann.

Zur Durchführung der Resektion wird vorzugsweise eine oszillierende Säge verwendet. Es ist angedacht, den Führungsschlitz / Sägeschlitz ca. 1 mm breit (+/- 0,1 bis 0,2 mm), und nach unten und/oder oben offen zu gestalten. Eine seitliche Führung des Sägeblatts ist in jedem Fall vorgegeben.

Die Resektionsschablone soll in erster Linie am Stück an den Unterkiefer angebracht werden können. Zusätzlich hierzu wird ergo dann ermöglicht, dass auch nur einzelne Segmente an den Unterkiefer angelegt werden. Über einen Steckmechanismus oder eine ähnliche Verbindungskonstellation werden das vordere Segment inklusive der rechten Seite bzw. das vordere Segment inklusive der linken Seite vom Rest abtrennbar gehalten.

Integrierte Bohrungen ermöglichen eine Fixierung der Schablone mittels Standardschrauben, die bspw. einen Durchmesser von 2,0 mm aufweisen, am Unterkiefer.

Eine angebrachte Mittellinienmarkierung im vorderen Segment wirkt sich vorteilhaft auf die Orientierung und genaue Ausrichtung der Resektionsschablone am Unterkiefer aus. Eine exakte Positionierung wird erleichtert.

Alle abnehmbaren Komponenten der Resektionsschablone werden zusätzlich Seiten-Markierungen erhalten, wie z.B. "R" für Rechts und "L" für Links. Eine Tumorentfernung bzw. Resektion des Unterkiefers erfolgt nach anatomischen Regionen (rechter Unterkieferkörper, Symphyse, linker Unterkieferkörper), d.h., wenn sich der Tumor bspw. mittig im Unterkieferkörper befindet, wird bis zum Unterkieferwinkel und zum Symphysenbereich resektiert.

Vorteilhafte Ausführungsformen sind in den Unteransprüchen beansprucht und werden nachfolgend näher erläutert.

So ist es von Vorteil, wenn die Positionierhilfe als eine Kimme-Korn-Kombination, eine Kerbe, eine Bohrung, ein Prisma, eine Farbmarkierung, ein Stift, eine Schraube oder eine Navigationspositionierung ausgebildet ist und/oder als eine Positionierflosse zum in Kontakt gelangen mit einem Abschnitt des Unterkieferknochens, etwa dem Symphysensegment, vorbereitete Positionierflosse ausgebildet ist.

Auch ist es von Vorteil, wenn die Positionierhilfe bzw. die Positionierflosse vom Zentralbauteil quer, vorzugsweise orthogonal absteht. Die Ausrichtung der Unterkieferresektionsschablone wird dadurch erleichtert.

Zweckmäßig ist es, wenn das Zentralbauteil als Rahmen ausgebildet ist. Die Sicht auf den Unterkiefer wird dann verbessert.

Die Handhabbarkeit wird verbessert, wenn der Rahmen zwei Horizontalstege besitzt, die vorzugsweise an deren distalen Enden über die beiden Trennwerkzeugführabschnitte / Sägeblattführabschnitte miteinander verbunden sind.

Ein besonderes Ausführungsbeispiel ist dadurch gekennzeichnet, dass die beiden Trennwerkzeugführabschnitte / Sägeblattführabschnitte und die beiden Horizontalstege zumindest in einer Frontalprojektion eine rechteckige Form besitzen. Der Zusammenbau der Einzelbauteile wird dann erleichtert.

Es ist auch von Vorteil, wenn die beiden Horizontalstege und die beiden Trennwerkzeugführabschnitte / Sägeblattführabschnitte als ein einheitliches und/oder einstückiges / integrales und/oder einmaterialiges Bauteil ausgeformt sind oder zumindest einer der Trennwerkzeugführabschnitte / Sägeblattführabschnitte oder beide Trennwerkzeugführabschnitte / Sägeblattführabschnitte jeweils ein Bestandteil eines von den Horizontalstegen lösbaren / verschieblichen / separaten Verschiebebauteils ist.

Wenn zumindest einer der Trennwerkzeugführabschnitte / Sägeblattführabschnitte einen Führungsschlitz / Sägeschlitz besitzt, der zum Führen eines Knochentrennwerkzeuges, wie eines Sägeblattes / einer Kreissäge / eines Lasers oder dergleichen, dimensioniert ist, so wird ein Verlaufen oder Verkanten des Knochentrennwerkzeuges verhindert.

Eine besonders gut wirkende Führung wird dadurch erreicht, wenn beidseitig des Führungsschlitzes / Sägeschlitzes eine zu diesem parallele Knochentrennwerkzeuganlagefläche ausgebildet ist.

Dabei ist es von Vorteil, wenn der Führungsschlitz / Sägeschlitz vollständig durch das ihn ausbildende Material hindurch ragt und zusätzlich in Verlängerung seiner Längserstreckung einseitig oder zweiseitig offen ausgestaltet ist.

Besonders bruchresistent ist die Unterkieferresektionsschablone dann, wenn das Führungsschlitz / Sägeschlitz beherbergende Material blockartig, vorzugsweise mit zueinander (nahezu) orthogonalen Oberflächen, ausgestattet ist und/oder einer der Horizontalstege rechteckbalkenartig ausgebildet ist, wobei die Horizontalstege bspw. geometrisch identisch ausgeformt sind.

Es ist von Vorteil, wenn im Bereich der Positionierflosse eine Markierung zur Positionierhilfe vorhanden ist, etwa nach Art einer Mittellinienmarkierung, eines (Durchgangs/Sack-)Loches, eines Vorsprunges, einer Vertiefung / Ausnehmung, eines Grates, einer Nut, einer Rille oder einer Riffelung. Wird in den Unterkiefer bspw. ein Schlitz eingebracht, dann kann der Grat dort einfach eingesetzt werden. Über einen Formschluss ist dann die Unterkieferresektionsschablone präzise ausrichtbar. Aber auch sonstige grafische Mittellinienmarkierungen helfen weiter.

Vorteilhaft ist es, wenn die Markierung als entlang der Längsachse der Flosse verlaufender Grat mit Spitzen, bspw. einem spitzen bzw. dreieckförmigen Querschnitt ausgebildet ist. Obwohl hier Nachteile bei der Fertigung zu erwarten sind, ist das Ausrichten der Schablonen zum Knochen bei einer derartigen Konfiguration besonders schnell und kippsicher möglich.
Die Sichtbarkeit wird verbessert, wenn die Markierung an einer Vorder- und/oder Oberseite nur eines Horizontalsteges oder beider Horizontalstege und/oder der Oberseite der Positionierflosse vorhanden ist und/oder an der Unterseite des Zentralbauteils.

Nicht unerwähnt bleiben sollte, dass der Grat auf der Oberseite der Positionierflosse vorhanden ist. Dadurch lässt sich ein effektives Handling betreiben.

Wenn die Positionierflosse von einem der beiden Horizontalstege, vorzugsweise nur dem unteren der beiden Horizontalstege senkrecht und vorzugsweise auch senkrecht zu den beiden Führungsschlitzen / Sägeschlitzen absteht, etwa als integraler Bestandteil des Horizontalsteges, so kann die Unterkieferresektionsschablone gut von unten angebracht werden.

Es hat sich bewährt, wenn von einem oder jedem der beiden Trennwerkzeugführabschnitte / Sägeblattführabschnitte auf der zentralbauteilabgewandten Seite ein Erweiterungsbauteil anschließt. Der zu behandelnde Raum kann dann erweitert werden.

Dabei ist es hilfreich, wenn das Erweiterungsbauteil als ein integraler Bestandteil des Zentralbauteils ausgebildet ist oder als ein lösbar an einer Verbindungsstelle ankoppelbares und separates Erweiterungsbauteil ausgebildet ist.

Ein vorteilhaftes Ausführungsbeispiel ist auch dadurch gekennzeichnet, dass die Verbindungsstelle zwei zueinander passende form- und/oder kraftschlüssig zusammenwirkende Koppelgeometrien einsetzt.

Gerade wenn die eine Koppelgeometrie einen Vorsprung ausformt und die andere Koppelgeometrie eine darauf abgestimmte Ausnehmung ausformt, lässt sich ein schnelles Zusammenstecken der Einzelbauteile bewirken.

Dabei hat es sich bewährt, wenn die Koppelgeometrien schwalbenschwanzartig zusammenwirken.

Ein vorteilhaftes Ausführungsbeispiel ist auch dadurch gekennzeichnet, dass die Ausnehmung als an einem Teilbereich entlang ihrer Längsachse offenes Sackloch ausgebildet ist.

Wenn ein Boden des Sackloches einen Anschlag für den Vorsprung definiert, so wird selbst in stressigen Situationen ein präziser Zusammenbau der Einzelteile der Unterkieferschablone erleichtert.

Die Resektion ist auch besonders gut durchführbar, wenn eine durch die Führungsschlitze / Sägeschlitze verlaufende Ebene, bei Messung auf einer Unterkiefer zugewandten Seite, einen Winkel von ca. 10° bis ca. 20°, vorzugsweise ca. 12° +/- 5° zur oberen und unteren Horizontalstrebe einnimmt.

Es ist von Vorteil, wenn das Erweiterungsbauteil einen Rundkörper besitzt, von dem ein Zusatztrennwerkzeugführabschnitt / Zusatzsägeblattführabschnitt distal, d.h. an einem freien Ende, absteht, ergo auf einer verbindungsstellenfernen Seite vorhanden ist.

Es hat sich bewährt, wenn im Grundkörper zumindest ein Durchgangsloch zum Aufnehmen einer Knochenschraube vorhanden ist. Ein Verrutschen der Unterkieferschablone während der Resektion, oder davor oder danach, wird verhindert.

Um ein gutes Heranziehen beim Einschrauben der Knochenschraube der Resektionsschablone an dem Unterkiefer zu erzwingen, ist es von Vorteil, wenn das Durchgangsloch zu der Oberfläche des Grundkörpers schräg verläuft, bspw. angestellt ist.

Um auch ein Verschwenken zu vermeiden, ist es dabei von Vorteil, wenn zwei Durchgangslöcher parallel zur Längsachse des Erweiterungsbauteils, vorteilsweise zu einer Außenkante des Erweiterungsbauteils versetzt, angeordnet sind.

Dabei hat es sich bewährt, wenn die Durchgangslöcher zur unteren Außenkante hin versetzt sind. Vorzugsweise sind die Durchgangslöcher aber im unteren Drittel des Erweiterungsbauteils angeordnet.

Genauer gesagt ist es also zweckmäßig, wenn die Durchgangslöcher von "vorne" nach "hinten" ansteigend verlaufen. "Vorne" ist dabei jener Bereich, der vom Patienten außerhalb angeordnet ist, wohingegen "hinten" dann auf der patienteninneren Seite angeordnet ist. "Unten" ist dabei durch die Schwerkraft bestimmt, genauso wie "oben".

Wenn eine Zentralachse eines Durchgangsloches zur vorderen oder hinteren, im Wesentlichen vertikal ausgerichteten Oberfläche, einen Winkel von ca. 20° +/- 5° einnimmt, so zieht sich beim Anschrauben der befestigenden Schrauben die Schablone gut passend an den Knochen heran.
Es hat sich bewährt, wenn der Zusatztrennwerkzeugführabschnitt / Zusatzsägeblattführabschnitt vom Grundkörper entfernbar ist, etwa über einen Verstellmechanismus. Die Flexibilität des Einsatzes bzw. die Einsatzmöglichkeiten werden dadurch erhöht.

Wenn der Zusatztrennwerkzeugführabschnitt / Zusatzsägeblattabschnitt einen Durchgangsschlitz zwischen zwei Führungsflächen besitzt, so kann auch dort die Präzision des vorzunehmenden Schnittes erhöht werden. Dabei ist vorzugsweise ein oberes oder unteres Ende des Durchgangsschlitzes offen zu lassen.

Ebenfalls hat es sich bewährt, wenn der Zusatztrennwerkzeugabschnitt / Zusatzsägeblattabschnitt ein von einem Block abstehenden Stab besitzt, der in einem bspw. offenen Kanal oder einer Rinne verstellbar gehalten ist.

Es ist von Vorteil, wenn der Stab im Kanal über ein Arretiermittel, wie eine Schraube, festlegbar ist.

Auch ist es von Vorteil, wenn am Erweiterungsbauteil ein Unterkieferauflagebock vorhanden ist.

Es ist wünschenswert, wenn der Unterkieferauflagebock dann (nahezu) senkrecht von der hinteren Oberfläche des Grundkörpers oder des Zusatztrennwerkzeugabschnittes / Zusatzsägeblattabschnittes absteht.

Wenn im Unterkieferbock ein Langloch ausgebildet ist, dessen längere Querachse durch die Längsrichtung des Unterkieferloches bestimmt ist, so wird ein einfaches Positionieren bei doch vorliegender Exaktheit möglich.

Von Vorteil ist es auch, wenn zwei Erweiterungsbauteile vorhanden sind, die zu einer Mittelebene spiegelsymmetrisch zueinander sind, wobei die Mittelebene jene Ebene ist, in der der Grat liegt und auf der die Horizontalstreben senkrecht stehen.

Ein vorteilhaftes Ausführungsbeispiel ist ferner dadurch gekennzeichnet, dass das Zentralbauteil mit seinen an beiden Enden abstehenden Erweiterungsbauteilen eine solche gebogene Form aufweist, die der Außenkontur eines durchschnittlichen menschlichen Unterkiefers (äquidistant) folgt

Es soll auch erwähnt werden, dass der Stab auf zumindest einer Oberfläche, etwa der Frontfläche, eine quer zur Längsrichtung des Stabes ausgerichtete Riffelung oder Rasterung besitzt.

Es ist von Vorteil, wenn die Unterkieferschablone aus Metall, etwa einer Titanlegierung, oder Kunststoff, etwa einem Polymer (vollständig) aufgebaut ist.

Hier sind bspw. Edelstahl, Titanlegierungen und Kunststoffe, etwa ABS-Kunststoffe denkbar.

Die Erfindung wird verwendet in einem Verfahren, in dem die Unterkieferresektionsschablone eingesetzt wird, um am Unterkiefer Knochenabschnitte zu entnehmen und/oder die Fibula-Knochenmaterialentnahme- und -verbringschablone eingesetzt wird, um Knochen vom Wadenbein zu entnehmen und/oder mittels der Fibula-Knochenmaterialentnahme- und -verbringschablone, die vom Wadenbein entnommenen Knochen in den Unterkieferbereich zu verbringen und dort zu implantieren.

Die Erfindung wird nachfolgend mit Hilfe einer Zeichnung näher erläutert, in der unterschiedliche Ausführungsformen dargestellt sind. Es zeigen:
- Fig. 1: eine Unterkieferresektionsschablone in einer perspektivischen Darstellung,
- Fign. 2 bis 5: die Unterkieferresektionsschablone der Fig. 1 in Anlage an einem Unterkiefer in unterschiedlichen Ansichten (von vorne, von der rechten Seite, von der linken Seite und von oben), jeweils leicht perspektivisch,
- Fign. 6 bis 9: weitere Darstellungen mit unterschiedlichen Unterkiefern, ähnlich der Darstellungen der Fign. 2 bis 5,
- Fig. 10: eine perspektivische Darstellung einer erfindungsgemäßen FibulaKnochenmaterialentnahme- und -verbringschablone,
- Fig. 11: die Fibula-Knochenmaterialentnahme- und -verbringschablone von hinten, d.h. von der Unterkieferseite,
- Fig. 12: -eine weitere Fibula-Knochenmaterialentnahme- undverbringschablone mit niedrigem Entnahmehilfsbügel,
- Fig. 13: eine Ansicht von oben auf die Fibula-Knochenmaterialentnahme- und -verbringschablone aus Fig. 12 in Anlage an eine Fibula mit bereits durchgeführten Schnitten,
- Fig. 14: die Fibula-Knochenmaterialentnahme- und -verbringschablone aus Fig. 13 mit entfernten Restknochenabschnitten, wobei die zu transplantierenden Knochenabschnitte an der Schablone befestigt sind,
- Fign. 15 bis 17: eine zweite Ausführungsform einer Fibula-Knochenmaterialentnahmeund -verbringschablone, mit einem höheren Entnahmehilfsbügel und mehrere Schlitze gemeinsam aufnehmenden Blöcken, wobei die Fig. 15 der Darstellung der Fig. 12 entspricht und die Fig. 16 einer Darstellung der Fig. 13 entspricht sowie die Fig. 17 einer Darstellung der Fig. 14 entspricht,
- Fig. 18: die Fibula-Knochenmaterialentnahme- und -verbringschablone gemäß der Erfindung in einer Verbringposition, bei der die zu transplantierenden Knochenstücke in die Form des zu ersetzenden oder zu reparierenden Unterkieferknochens verbracht sind,
- Fign. 19 bis 20: eine weitere Darstellung der erfindungsgemäßen Fibula-Knochenmaterialentnahme- und -verbringschablone in Kontakt mit einem Wadenbein / einer Fibula in einer Ansicht von vorne (Fig. 19), von der Seite (Fig. 20) und in einer perspektivischen Ansicht (Fig. 21),
- Fign. 22 bis 24: eine weitere Darstellung des am Fibula-Knochen angebrachten Fibula-Knochenmaterialentnahme- und -verbringschablone, in mehreren den Fign. 19 bis 21 entsprechenden Darstellungsarten, wobei die zu entnehmenden Knochen dargestellt sind,
- Fign. 25 bis 27: die entnommenen Knochenstücke an der angebrachten FibulaKnochenmaterialentnahme- und -verbringschablone in zu den Fign. 22 bis 24 vergleichbaren Darstellungsarten,
- Fign. 28 bis 30: den zum ersten Entnahmehilfsbügel geometrisch veränderten Implantierhilfsbügels, mittels dessen die entnommenen Knochenstücke in eine unterkieferähnliche Form gebracht werden und somit transplantierfähig vorbereitet werden,
- Fign. 31 bis 33: die in Position gebrachten Fibula-Knochenstücke im an den Restunterkiefer eingesetzten Zustand in einer Ansicht von oben, von vorne und einer perspektivischen Darstellung,
- Fign. 34 bis 38: die transplantierten Knochen im Unterkieferbereich in unterschiedlichen räumlichen Darstellungen (von oben, von vorne, perspektivisch von vorne, perspektivisch von der Seite rechts und perspektivisch von der Seite links).

Die Figuren sind lediglich schematischer Natur und dienen nur dem Verständnis der Erfindung. Die gleichen Elemente sind mit denselben Bezugszeichen versehen. Merkmale der einzelnen Ausführungsbeispiele können untereinander ausgetauscht werden.

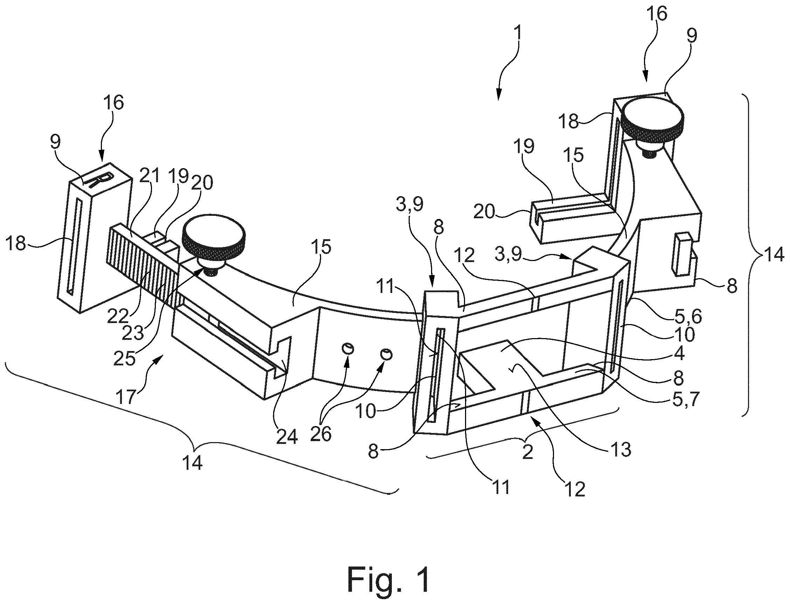

In Fig. 1 ist eine Unterkieferresektionsschablone 1 dargestellt. Die Unterkieferresektionsschablone 1 besitzt ein Zentralteil / Zentralbauteil 2. Dieses Zentralbauteil 2 ist zum Verbrachtwerden an einem Symphysensegment eines Unterkiefers vorbereitet. Das Zentralbauteil 2 weist an beiden äußeren Enden, wobei die Enden die Längsachse definieren, zwei Trennwerkzeugführabschnitte / Sägeblattführabschnitte 3 auf. Zwischen den beiden Trennwerkzeugführabschnitten / Sägeblattführabschnitten 3 ist eine Positionierflosse 4 vorhanden. Die Trennwerkzeugführabschnitte müssen nicht in Kontakt gelangen mit einem Trennwerkzeug, wohingegen die dazu vergleichbaren Sägeblattführabschnitte durchaus zum physischen in Kontaktgelangen mit einem Trennwerkzeug, wie einem Fräser, einem Sägeblatt oder einem anderen metallischen spanabhebenden Werkzeug. Die Positionierhilfe muss auch nicht zwingen in physischem Kontakt oder gar in Anschlag mit dem Knochen gelangen, sondern kann beispielsweise auch nur optische Mittel zur Ausrichtung nutzen. Bei Ausgestaltung der Positionierhilfe als Positionierflosse ist der ein physisches in Kontaktgelangen mit dem Knochen aber wünschenswert. Diese Positionierhilfe / Positionierflosse 4 ist nicht zwingend für bestimmte Ausführungsformen der Erfindung nötig.

Die Positionierflosse 4 steht hier jedenfalls senkrecht auf einer Ebene durch das Zentralbauteil 2 in Richtung des Unterkiefers von einem horizontal Steg 5 ab. Es gibt einen oberen Horizontalsteg 6 und einen unteren Horizontalsteg 7. Die Positionierflosse 4 steht vom unteren Horizontalsteg 7 ab. Sie ist im hier vorliegenden Ausführungsbeispiel plattenartig ausgebildet, kann aber auch stiftartig ausgebildet sein, etwa mit kreisrundem, elliptischem oder polygonalem Querschnitt.

An distalen Enden 8 der Horizontalstege 5 sind die Trennwerkzeugführabschnitte / Sägeblattführabschnitte 3 nach Art von Blöcken 9 einstückig und einmaterialig angebracht. In den Blöcken 9 sind Führungsschlitze/Sägeschlitze 10 vorhanden. Pro Trennwerkzeugführabschnitt / Sägeblattführabschnitt 3 ist ein Führungsschlitz / Sägeschlitz 10 vorhanden. Der Führungsschlitz / Sägeschlitz 10 befindet sich zwischen zwei parallel zueinander verlaufenden vertikal ausgerichteten Knochentrennwerkzeuganlageflächen 11.

Am unteren Horizontalsteg 7 vorne und/oder unten ist eine Markierung 12 als Mittellinienmarkierung ausgebildet, genauso wie am oberen Horizontalsteg 6 vorne und/oder oben.

Statt der Mittellinienmarkierung ist auch auf jener dem oberen Horizontalsteg 6 zugewandten Oberfläche 13 der Positionierflosse 4 ein in Längsrichtung der Positionierflosse 13, also vom unteren Horizontalsteg 7 in Richtung des Unterkiefers verlaufender Grat (nicht dargestellt) anbringbar. Dieser Grat kann dann in eine in den Knochen gebrachte Kerbe eingreifen und positionierend wirken.

Beiderseits des Zentralbauteils 2 steht jeweils ein Erweiterungsbauteil 14 ab. Die beiden Erweiterungsbauteile 14 sind hier als integrale Bestandteile des Zentralbauteils 2 auskonstruiert. Allerdings kann ein Erweiterungsbauteil 14 oder beide Erweiterungsbauteile 14 auch lösbar am Zentralbauteil 2, nämlich auf der Außenseite je eines Blockes 9 angekoppelt werden. Dazu sind dann entsprechende Verbindungsstellen mit Koppelgeometrien, wie Vorsprüngen und Ausnehmungen, etwa nach Art von Schwalbenschwanzkonfigurationen, bspw. unter Ausbildung eines Bodens und eines Anschlages gestaltbar.

Jedenfalls weist jedes Erweiterungsbauteil 14 einen Grundkörper 15 auf, an dessen Ende jeweils ein Zusatzsägeblattführabschnitt 16 vorhanden ist. Dabei ist ein Verstellmechanismus 17 eingesetzt, um eine Verschiebbarkeit des Zusatztrennwerkzeugführabschnittes / Zusatzsägeblattführabschnittes 16, nach Art eines weiteren Blockes vom Grundkörper 15 zu gewährleisten. Auch in den blockartigen Zusatztrennwerkzeugführabschnitten / Zusatzsägeblattführabschnitten 16 sind Durchgangsschlitze 18 vorhanden, die ähnlich oder identisch zu den Schlitzen / Sägeschlitzen 10 in den Trennwerkzeugführabschnitten / Sägeblattführabschnitten 3 ausgestattet sind.

Von den Enden des Grundkörpers 15, etwa im Bereich des Verstellmechanismus 17 oder aber von den Zusatztrennwerkzeugführabschnitten / Zusatzsägeblattführabschnitten 16 steht ein Unterkieferauflagebock 19 mit einem Langloch ab. Das Langloch ist nicht dargestellt. Im hier vorgestellten Ausführungsbeispiel wird eine Schlitzverlängerung 20 stattdessen genutzt. Das Langloch kann also die Schlitzverlängerung 20 ersetzen, die Längsachse des als Durchgangsloch ausgebildeten Langlochs ist vorzugweise in Richtung des Schlitzes der Schlitzverlängerung 20 ausgerichtet.

Der Verstellmechanismus weist einen Stab 21 auf, auf dessen Frontfläche / Frontoberfläche 22 eine Riffelung 23 vorhanden ist. Der Stab 21 greift in einen offen konstruierten Kanal 24 und wird durch ein als Feststellschraube ausgebildetes Arretiermittel 25 lösbar in Position gehalten. Es gibt in jedem Erweiterungsbauteil 14 zumindest zwei Durchgangslöcher 26 für die Befestigung der Unterkieferresektionsschablone 1 mittels Schrauben am hier nicht dargestellten Unterkiefer.

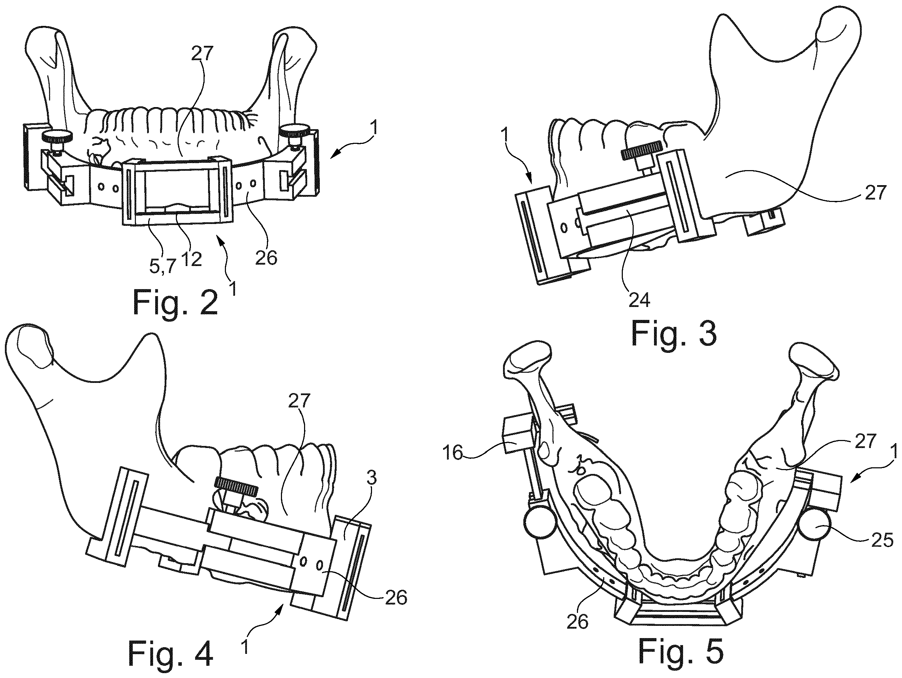

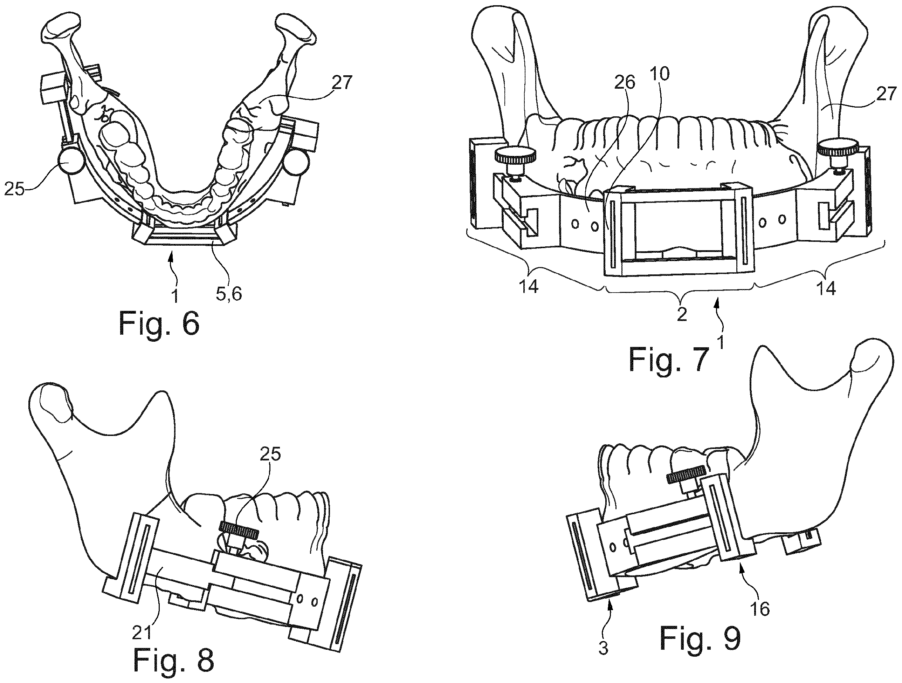

In den Fign. 2 bis 9 ist die erfindungsgemäße Unterkieferresektionsschablone in unterschiedlichen Positionen an einem Unterkiefer 27 dargestellt.

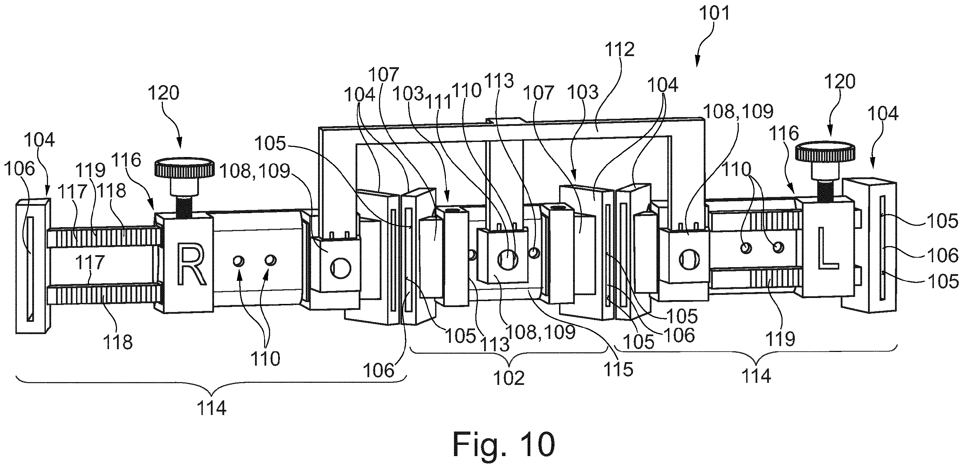

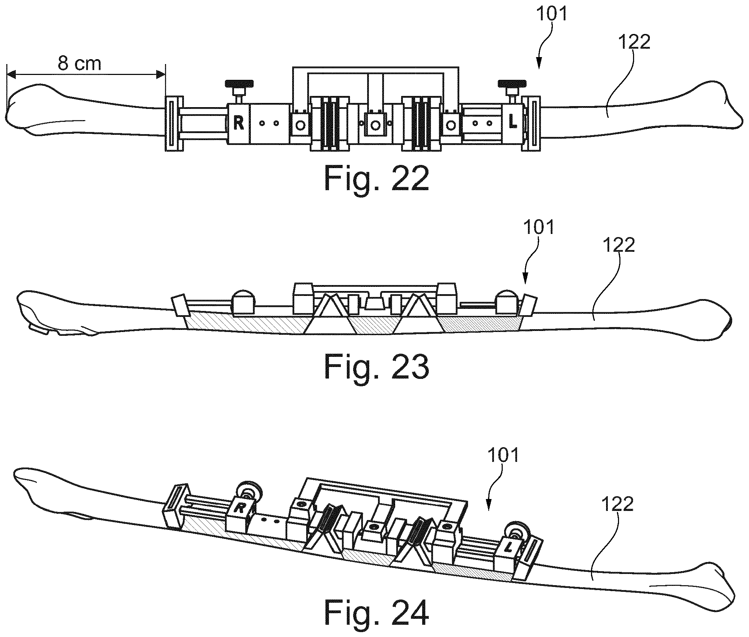

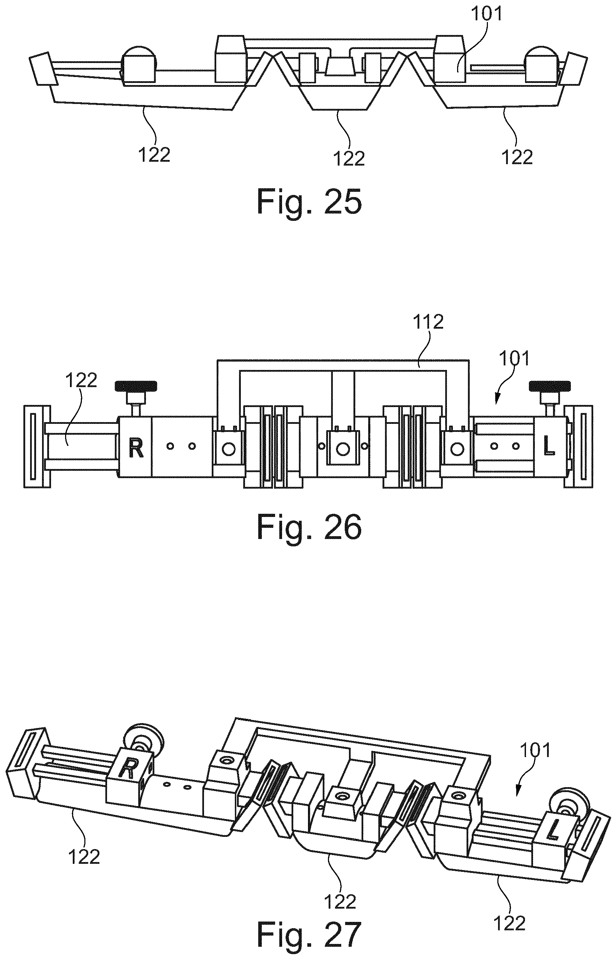

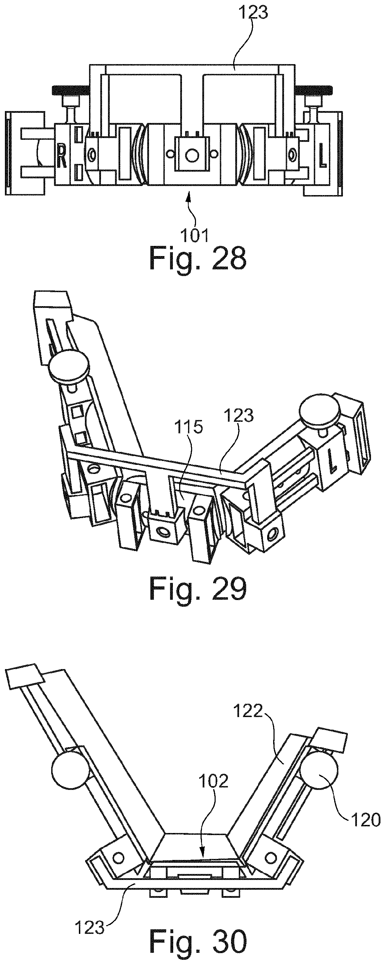

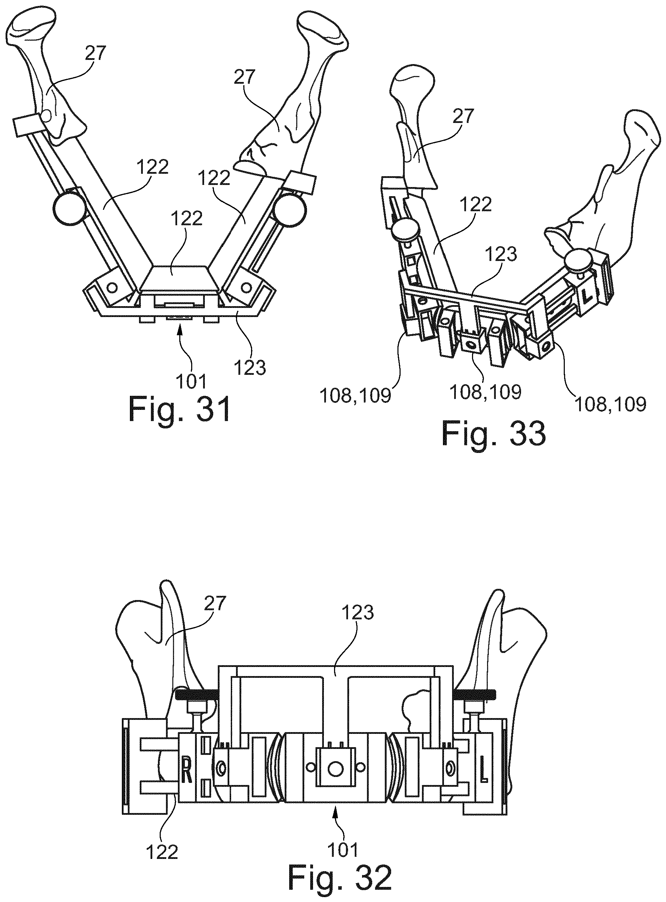

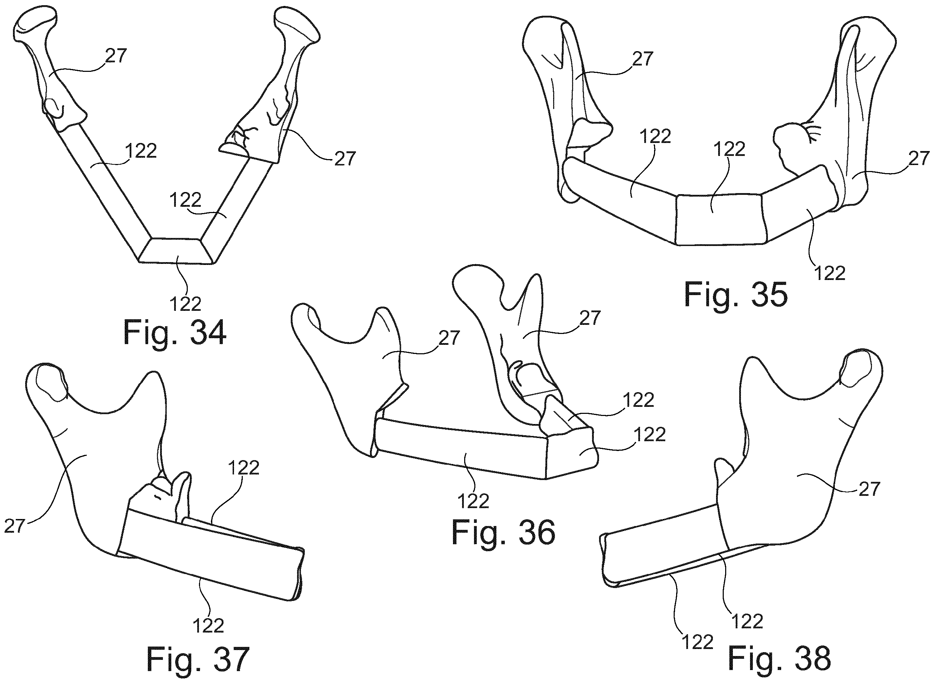

In den Fign. 10 bis 38 ist nun nachfolgend vermehrt der Augenmerk auf eine erfindungsgemäße Fibula-Knochenmaterialentnahme- und -verbringschablone bzw. den an einem Fibula-Knochen entnommenen Knochen und zu einem in den Unterkiefer zu transplantierenden Knochenstücken Augenmerk gelegt.

So ist in Fig. 10 eine erfindungsgemäße Fibula-Knochenmaterialentnahme- und - verbringschablone 101 dargestellt. Diese Fibula-Knochenmaterialentnahme- und - verbringschablone 101 weist ein Mittelteil 102 auf. An dessen distalen Enden 103 ist jeweils ein Knochentrennwerkzeugführabschnitt 104 vorhanden. Diese Knochentrennwerkzeugführabschnitte 104 können als Backen oder Blöcke ausgebildet sein und entweder einstückig mit dem Mittelteil bzw. eines Grundkörpers / Basiskörpers des Mittelteils 102 verbunden sein oder verschiebbar angekoppelt sein. Zumindest einer der Knochenwerkzeugführabschnitte 104 sollte aber entfernbar und heranschiebbar gelagert sein.

Jeder Knochentrennwerkzeugführabschnitt 104 weist zwischen zwei Vertikalflächen 105 einen Führungsschlitz 106 auf. Diese Führungsschlitze 106 sind auf der Vorderund Rückseite offen / durchlässig ausgestaltet. Es ist jeder Führungsschlitz 106 bis auf die länglichen Öffnungen in der Vorder- und Rückseite vollständig von Material umgeben. Allerdings kann ein Führungsschlitz 106 unten und/oder oben offen sein.

Für eine Verschiebbarkeit eines Knochentrennwerkzeugführungsabschnitts 104 bietet sich das Vorhalten eines Balkens 107 an. Der Balken 107 ist in einer ihn umfassenden Führungsbahn verschieblich gelagert. Eine Fixierschraube kann dabei zum Befestigen des Balkens 107 eingesetzt sein.

In der Mitte des Mittelteils 102 befindet sich eine Bügelaufnahmevorrichtung 108. Die Bügelaufnahmevorrichtung 108 ist hier als Klammer 109 ausgebildet und weist ein Durchgangsloch 110 auf, in das ein konvexer Federabschnitt 111 eines Entnahmehilfsbügels 112 eingreift. Links und rechts der Klammer 109 befindet sich jeweils ein Aufnahmeloch 113, um eine Knochenschraube aufzunehmen, mittels derer ein Anbringen an einem Fibula-Knochen durchgeführt werden kann. Die Aufnahmelöcher 113 sind nach Art von Bohrungen ausgebildet.

Beiderseits des Mittelteils 102 schließt jeweils ein Ergänzungsbauteil 114 an. Zwischen dem Ergänzungsbauteil 114 ist somit ein Zentralkörper 115 des Mittelteils 102 angeordnet.

Jedes Ergänzungsbauteil 114 weist einen weiteren Knochentrennwerkzeugführabschnitt 104 auf. Jeder dieser Knochentrennwerkzeugführabschnitte 104 der Ergänzungsbauteile 114 ist dann über einen Verschiebemechanismus 116 verschieblich gehalten. Dabei werden jeweils zwei Führungsstege 117 eingesetzt, die beide auf ihrer Frontseite 118 Rippen, Kerben oder Rasten besitzen, um eine Rasterung 119 auszubilden. Es sind wiederum Bügelaufnahmevorrichtungen 108 nach Art von Klammern 109 vorhanden, in die Enden des Entnahmehilfsbügels 112 eingreifen. Die Verbindung zwischen dem Entnahmehilfsbügel 112 und den Klammern 109 ist ähnlich oder identisch wie schon vorher ausgeführt ausgebildet.

In jedem der Ergänzungsbauteile 114 sind ebenfalls Durchgangslöcher 110 vorhanden, um eine Befestigung mit Knochen über Schrauben zu ermöglichen. Der Entnahmehilfsbügel 112 ist abnehmbar zur Schablonenfixierung. Es sind Feststellschrauben 120 zur Fixierung des flexiblen Wegeschlitzes eingesetzt.

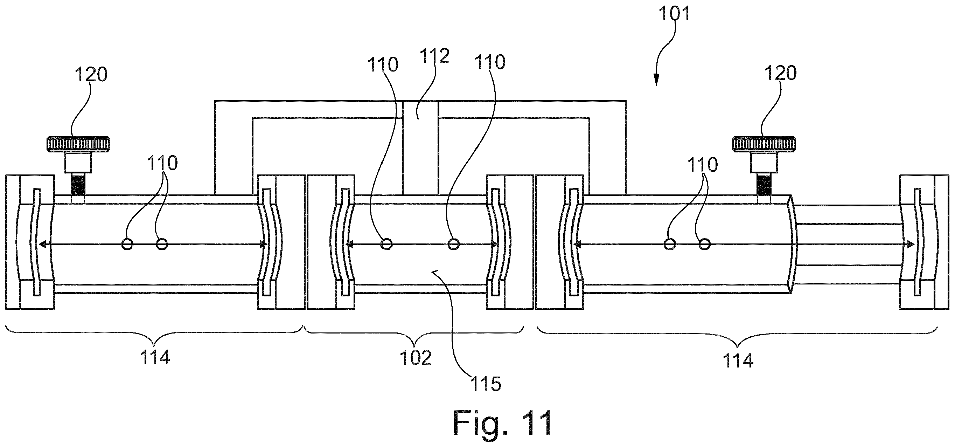

Während in der Fig. 10 die Fibula-Knochenmaterialentnahme- und -verbringschablone im Wesentlichen von vorne gezeigt ist, ist diese in der Fig. 11 im Wesentlichen von hinten, also vom Fibula-Knochen aus gesehen, dargestellt. Man sieht also die Rückseite. Die Länge der Ergänzungsbauteile 114 sollte möglichst zwischen 45,2 mm und 66,2 mm variierbar sein. Die Sequenzlänge des Mittelteils 102 sollte möglichst 30 mm betragen und kann fix sein.

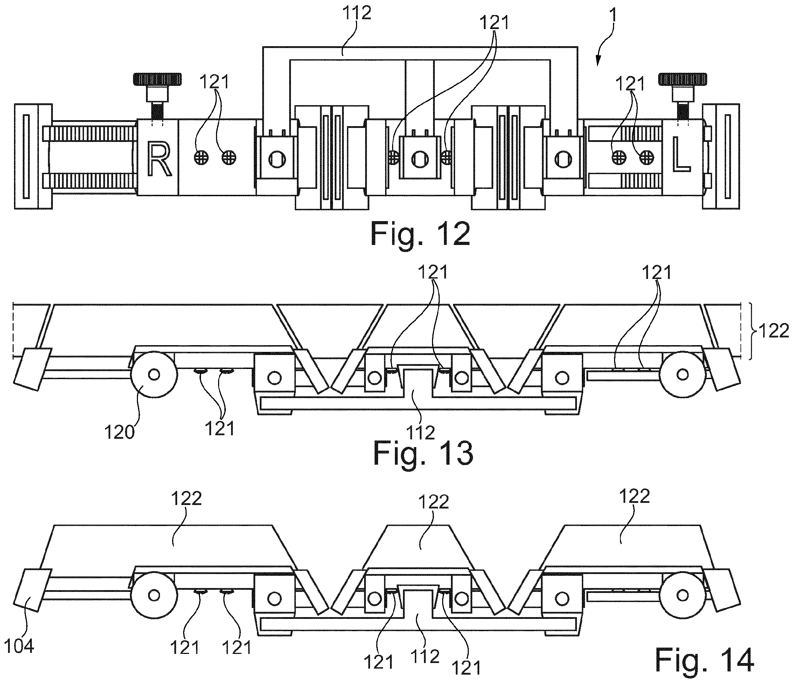

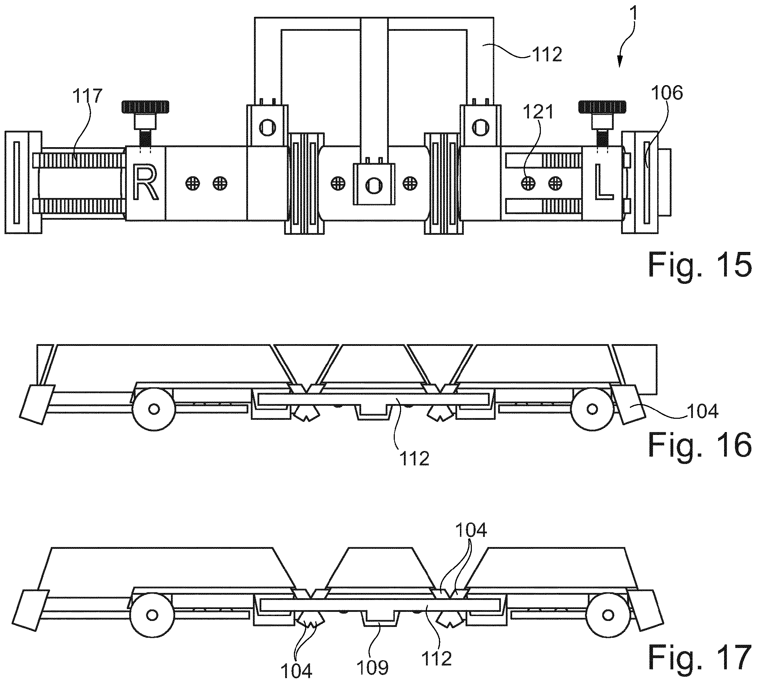

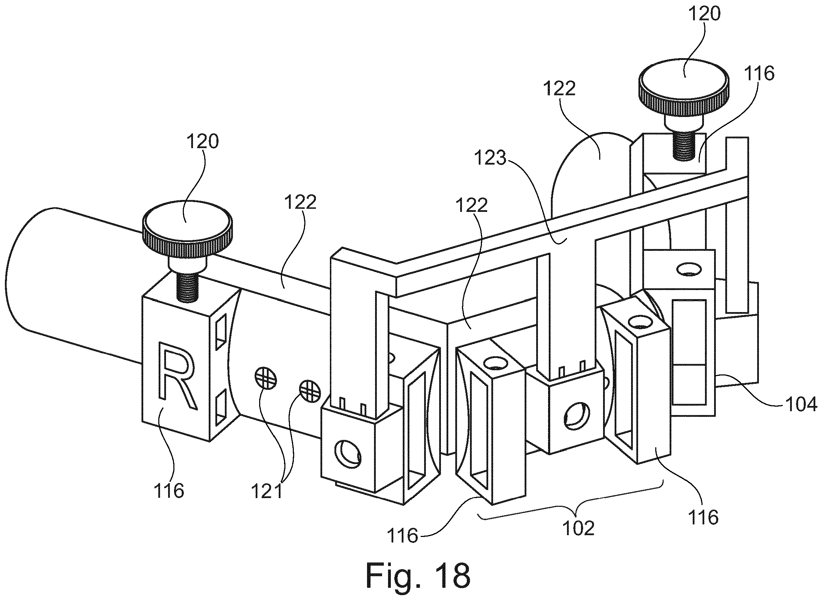

In den Fign. 12 und 15 sind bereits Knochenschrauben 121 eingesetzt, um, wie in den Fign. 13 und 14 und 16 sowie 17 die Befestigung an Teilen eines Fibula-Knochens 122 zu bewerkstelligen. In Fig. 18 ist ein geometrisch vom Entnahmehilfsbügel 112 leicht veränderter Implantierhilfsbügel 123 eingesetzt. Die einzelnen Abschnitte des Fibula-Knochens 122 werden dann komplett neu angeordnet, ähnlich in der räumlichen Lage wie von der Unterkieferresektionsschablone 1 vordefiniert, vorzugsweise identisch. Die distal äußersten Knochentrennwerkzeugführabschnitte 104 sind dabei entfernt worden. Dies gilt auch für die Knochentrennwerkzeugführabschnitte 104 des Mittelteils 102. Die Sägeschlitze bzw. Führungsschlitze 106 wurden entfernt, um die Segmente "Stoß auf Stoß" setzen zu können.

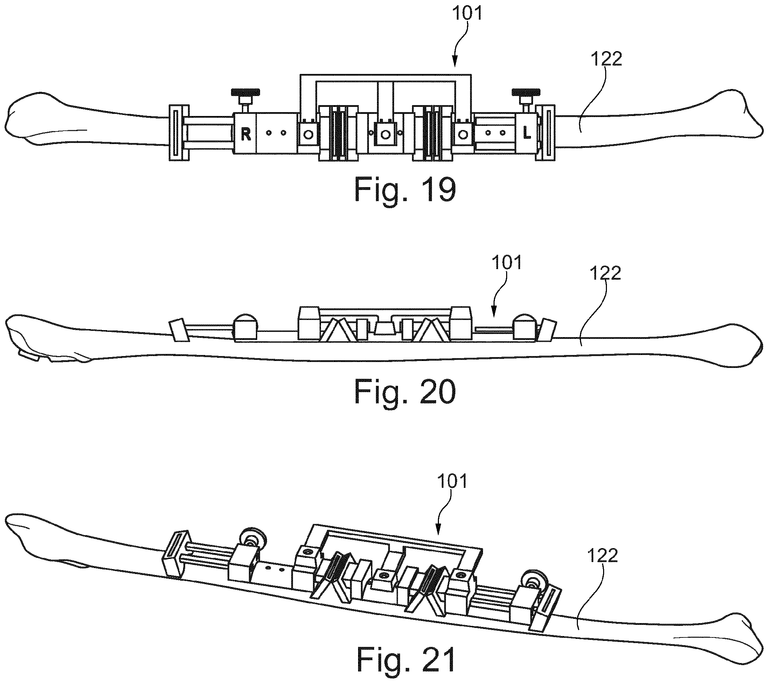

Der Vorgang des Aufsetzens, Schneidens und Entnehmens sowie nachfolgenden Zusammensetzens ist den Fign. 19 bis 38 gut zu entnehmen.

### Bezugszeichenliste

- 1: Unterkieferresektionsschablone
- 2: Zentralteil / Zentralbauteil
- 3: Trennwerkzeugführabschnitt / Sägeblattführabschnitt
- 4: Positionierhilfe / Positionierflosse
- 5: Horizontalsteg
- 6: oberer Horizontalsteg
- 7: unterer Horizontalsteg
- 8: distales Ende des Horizontalsteges
- 9: Block
- 10: Sägeschlitz
- 11: Trennwerkzeuganlagefläche
- 12: Markierung
- 13: Oberfläche der Positionierflosse
- 14: Erweiterungsbauteil
- 15: Grundkörper
- 16: Zusatztrennwerkzeugführabschnitt / Zusatzsägeblattführabschnitt
- 17: Verstellmechanismus
- 18: Durchgangsschlitz
- 19: Unterkieferauflagebock
- 20: Schlitzverlängerung
- 21: Stab
- 22: Frontoberfläche
- 23: Riffelung
- 24: Kanal
- 25: Arretiermittel
- 26: Durchgangsloch
- 27: Unterkiefer
- 101: Fibula-Knochenmaterialentnahme- und -verbringschablone
- 102: Mittelteil
- 103: Ende
- 104: Knochentrennwerkzeugführabschnitt
- 105: Vertikalfläche
- 106: Führungsschlitz
- 107: Balken
- 108: Bügelaufnahmevorrichtung
- 109: Klammer
- 110: Durchgangsloch
- 111: Federabschnitt
- 112: Entnahmehilfsbügel
- 113: Aufnahmeloch
- 114: Ergänzungsbauteil
- 115: Zentralkörper
- 116: Verschiebemechanismus
- 117: Führungssteg
- 118: Frontseite
- 119: Rasterung
- 120: Verstellschraube
- 121: Knochenschraube
- 122: Fibula-Knochen
- 123: Implantierhilfsbügel

## Patentansprüche

1. Fibula-Knochenmaterialentnahme- und -verbringschablone (101) mit einem Mittelteil (102), das einen Zentralkörper (115) besitzt, an dessen Enden (103) je ein Knochentrennwerkzeugführabschnitt (104) vorhanden ist, wobei zumindest einer der Knochentrennwerkzeugführabschnitte (104) vom Mittelteil (102) wegbewegbar gelagert ist, **dadurch gekennzeichnet, dass** eine Bügelaufnahmevorrichtung (108) vorhanden ist, in die ein Entnahmehilfsbügel (112) eingesteckt ist.

2. Fibula-Knochenmaterialentnahme- und -verbringschablone (101) nach Anspruch 1, **dadurch gekennzeichnet, dass** der Knochentrennwerkzeugführabschnitt (4) einen zwischen zwei Vertikalflächen (105) ausgebildeten Führungsschlitz (106) besitzt, der auf dessen Vorder- und Rückseite offen ist.

3. Fibula-Knochenmaterialentnahme- und -verbringschablone (101) nach Anspruch 2, **dadurch gekennzeichnet, dass** der Führungsschlitz (106) auf dessen Unterseite oder Oberseite offen ausgestaltet ist.

4. Fibula-Knochenmaterialentnahme- und - verbringschablone (101) nach einem der Ansprüche 1 bis 3, **dadurch gekennzeichnet, dass** von dem zum Mittelteil (102) separaten Knochentrennwerkzeugführabschnitt (104) ein Balken (107) absteht, der in einer ihn umfassenden Führungsbahn entlang ihrer Längsrichtung verschieblich gelagert ist.

5. Fibula-Knochenmaterialentnahme- und -verbringschablone (101) nach Anspruch 4, **dadurch gekennzeichnet, dass** in die Führungsbahn eine Fixierschraube ragt, die zum Befestigen des Balkens (107) ausgelegt ist.

6. Fibula-Knochenmaterialentnahme- und -verbringschablone (101) nach einem der Ansprüche 1 bis 5, **dadurch gekennzeichnet, dass** in der Bügelaufnahmevorrichtung (108) ein Loch vorhanden ist, das zum arretierenden Aufnehmen eines entnahmehilfsbügelfesten Federabschnittes (111) vorbereitet ist.

7. Fibula-Knochenmaterialentnahme- und -verbringschablone (101) nach einem der Ansprüche 1 bis 6, **dadurch gekennzeichnet, dass** beiderseits einer Klammer (109) ein Aufnahmeloch (113) für eine Knochenschraube vorhanden ist.

8. Fibula-Knochenmaterialentnahme- und -verbringschablone (101) nach Anspruch 7, **dadurch gekennzeichnet, dass** das Aufnahmeloch (113) eine Symmetrieachse besitzt, die quer zu einer den Zentralkörper (115) aufnehmenden Zentralebene ausgerichtet ist.

9. Fibula-Knochenmaterialentnahme- und -verbringschablone (101) nach einem der Ansprüche 1 bis 8, **dadurch gekennzeichnet, dass** auf einem oder jedem der beiden Knochentrennwerkzeugführabschnitte (104) ein Ergänzungsbauteil (114) auf der dem Zentralkörper (115) abgewandten Seite vorhanden ist.

## Claims

1. A fibula bone material removal and transfer template (101) comprising a center part (102) with a central body (115), each end (103) of which has a bone separating tool guide portion (104), wherein at least one of the bone separating tool guide portions (104) is mounted such that it can be moved away from the center part (102), **characterized in that** a bracket receiving device (108) is provided into which an auxiliary resection bracket (112) is inserted.

2. The fibula bone material removal and transfer template (101) according to claim 1, **characterized in that** the bone separating tool guide portion (4) has a guide slit (106) formed between two vertical surfaces (105) which is open on the front and rear sides thereof.

3. The fibula bone material removal and transfer template (101) according to claim 2, **characterized in that** the guide slit (106) is configured to be open on the lower side or upper side thereof.

4. The fibula bone material removal and transfer template (101) according to one of the claims 1 to 3, **characterized in that** a beam (107), which is movably supported in a guide path encompassing the same along its longitudinal direction, projects from the bone separating tool guide portion (104) separate from the center part (102).

5. The fibula bone material removal and transfer template (101) according to claim 4, **characterized in that** a fixing screw designed for securing the beam (107) protrudes into the guide path.

6. The fibula bone material removal and transfer template (101) according to one of claims 1 to 5, **characterized in that** a hole prepared for receiving a spring portion (111) fixed to the auxiliary resection bracket by locking is provided in the bracket receiving device (108).

7. The fibula bone material removal and transfer template (101) according to one of claims 1 to 6, **characterized in that** a receiving hole (113) for a bone screw is provided on both sides of a clip (109).

8. The fibula bone material removal and transfer template (101) according to claim 7, **characterized in that** the receiving hole (113) has an axis of symmetry which is orientated transversely to a central plane receiving the central body (115).

9. The fibula bone material removal and transfer template (101) according to one of claims 1 to 8, **characterized in that** a supplementary component (114) is present on one or each of the two bone separating tool guide portions (104) on the side facing away from the central body (115).

## Revendications

1. Gabarit de prélèvement et de transfert de matériau d'os fibulaire (101) avec une partie médiane (102) qui possède un corps central (115), aux extrémités duquel (103) une section de guidage d'outil de séparation d'os (104) est à chaque fois présente, dans lequel au moins une des sections de guidage d'outil de séparation d'os (104) est logée de manière mobile à partir de la partie médiane (102), **caractérisé en ce qu'**un dispositif de réception d'étrier (108) est présent, dans lequel un étrier d'aide au prélèvement (112) est enfiché.

2. Gabarit de prélèvement et de transfert de matériau d'os fibulaire (101) selon la revendication 1, **caractérisé en ce que** la section de guidage d'outil de séparation d'os (4) possède une fente de guidage (106) réalisée entre deux surfaces verticales (105) qui est ouverte sur son côté avant et arrière.

3. Gabarit de prélèvement et de transfert de matériau d'os fibulaire (101) selon la revendication 2, **caractérisé en ce que** la fente de guidage (106) est configurée de manière ouverte sur son côté inférieur ou côté supérieur.

4. Gabarit de prélèvement et de transfert de matériau d'os fibulaire (101) selon l'une quelconque des revendications 1 à 3, **caractérisé en ce qu'**une barre (107) dépasse de la section de guidage d'outil de séparation d'os (104) séparée par rapport à la partie médiane (102), laquelle est logée de manière mobile dans une bande de guidage la comprenant le long de son sens longitudinal.

5. Gabarit de prélèvement et de transfert de matériau d'os fibulaire (101) selon la revendication 4, **caractérisé en ce qu'**une vis de fixation pénètre dans la bande de guidage, laquelle est conçue pour la fixation de la barre (107).

6. Gabarit de prélèvement et de transfert de matériau d'os fibulaire (101) selon l'une quelconque des revendications 1 à 5, **caractérisé en ce qu'**un trou est présent dans le dispositif de réception d'étrier (108), lequel est préparé pour la réception par arrêt d'une section de ressort (111) fixée à l'étrier d'aide au prélèvement.

7. Gabarit de prélèvement et de transfert de matériau d'os fibulaire (101) selon l'une quelconque des revendications 1 à 6, **caractérisé en ce qu'un** trou de réception (113) pour une vis d'os est présent de part et d'autre d'une attache (109).

8. Gabarit de prélèvement et de transfert de matériau d'os fibulaire (101) selon la revendication 7, **caractérisé en ce que** le trou de réception (113) possède un axe de symétrie qui est orienté transversalement à un plan central recevant le corps central (115).

9. Gabarit de prélèvement et de transfert de matériau d'os fibulaire (101) selon l'une quelconque des revendications 1 à 8, **caractérisé en ce qu'**un composant complémentaire (114) est présent sur une ou chacune des deux sections de guidage d'outil de séparation d'os (104) sur le côté éloigné du corps central (115).
